(19) **Europäisches Patentamt / European Patent Office / Office européen des brevets**

(11) **EP 3 012 455 B1**

(12) **EUROPEAN PATENT SPECIFICATION**

(45) Date of publication and mention
of the grant of the patent:
**28.10.2020 Bulletin 2020/44**

(21) Application number: **14813040.4**

(22) Date of filing: **20.06.2014**

(51) Int Cl.:
*F04B 49/10* (2006.01)     *A61M 1/00* (2006.01)
*A61M 1/14* (2006.01)     *F04B 9/04* (2006.01)
*F04B 17/00* (2006.01)     *F04B 19/22* (2006.01)
*F04B 51/00* (2006.01)     *F04B 53/10* (2006.01)
*F04B 53/14* (2006.01)     *F04B 49/06* (2006.01)
*F04B 49/22* (2006.01)

(86) International application number:
**PCT/JP2014/066436**

(87) International publication number:
**WO 2014/203994 (24.12.2014 Gazette 2014/52)**

(54) **RECIPROCATING PUMP**

KOLBENPUMPE

POMPE ALTERNATIVE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.06.2013 JP 2013130912**

(43) Date of publication of application:
**27.04.2016 Bulletin 2016/17**

(73) Proprietor: **Nikkiso Co., Ltd.
Tokyo 150-6022 (JP)**

(72) Inventors:
• **MENJOH, Yuya
Makinohara-shi
Shizuoka 421-0496 (JP)**

• **YAMADA, Satoaki
Makinohara-shi
Shizuoka 421-0496 (JP)**
• **SUZUKI, Hiroaki
Makinohara-shi
Shizuoka 421-0496 (JP)**

(74) Representative: **Wunderlich & Heim
Patentanwälte
Partnerschaftsgesellschaft mbB
Irmgardstrasse 3
81479 München (DE)**

(56) References cited:
**EP-A1- 0 183 295     EP-A1- 2 505 836
EP-A1- 2 550 984     JP-A- H0 610 844
JP-A- H07 174 659     JP-A- 2003 284 772
JP-B2- 4 149 822     US-A- 4 832 575**

**Description**

Technical Field

The present invention relates to a reciprocating pump that includes a reciprocating device which is able to reciprocate in a pump chamber by a drive device, performs suction of a liquid from a suction port into the pump chamber due to the reciprocating, and enables the liquid to be discharged from a discharge port.

Background Art

In a blood purification device such as a dialyzer which is used during hemodialysis treatment, a dialysate supplying line for supplying dialysate is connected with a dialysate discharging line for discharging dialysis effluent. The dialysate supplying line and the dialysate discharging line are configured to extend from a dialysis device such that the lines are connected with a blood purification device and to supply dialysate to the blood purification device and to discharge dialysate (dialysis effluent) from the blood purification device.

In the dialysis device, a reciprocating pump (duplex pump) is disposed between the dialysate supplying line and the dialysate discharging line to connect both lines. For example, as disclosed in PTL 1, the reciprocating pump includes a housing in which a plunger is accommodated in a reciprocating manner, a liquid supply-side pump chamber and a liquid drainage-side pump chamber which are formed in the housing and are separated due to the plunger, and a motor for reciprocating the plunger.

In addition, in the configuration, valve devices such as a check valve are provided on a suction port side and on a discharge port side, respectively, in the liquid supply-side pump chamber and the liquid drainage-side pump chamber. When the motor is driven such that the plunger is caused to reciprocate, the dialysate is discharged from the liquid supply-side pump chamber and is supplied to the blood purification device and the dialysate (dialysis effluent) from the blood purification device can be suctioned to the liquid drainage-side pump chamber and can be discharged to the outside.

Incidentally, as disclosed in PTL 2, for example, a reciprocating pump is proposed, in which, in order to monitor shutout states (liquid-tight degrees of closed states) of the valve device on the suction port side and the valve device on the discharge port side, a transmitting electrode unit is formed in the pump chamber and a receiving electrode unit is formed in each of the valve device on the suction port side and the valve device on the discharge port side such that current is applied between the transmitting electrode unit and the receiving electrode units. According to the reciprocating pump, current application between the transmitting electrode unit and the receiving electrode units is detected and thereby, it is possible to monitor the shutout states (liquid-tight degrees of closed states) of the valve device on the suction port side and the valve device on the discharge port side.

EP 2 505 836 A1 describes a reciprocation pump with a supply liquid-side pumping chamber and a waste liquid-side pumping chamber having a reciprocating means that enables a liquid to be supplied to an objective apparatus.

According to EP 0 183 295 A1 the delivery rate of a positive displacement pump that comprises a piston driven with reciprocating movement in a chamber is measured.

In EP 2 550 984 A1 a blood purification apparatus is depicted that comprises a blood pump and a substitution pump that supplies a dialysate.

Citation List

Patent Literature

   PTL 1: Japanese Unexamined Patent Application Publication No. 2003-284772
   PTL 2: Japanese Unexamined Patent Application Publication No. 7-174659

Summary of Invention

Technical Problem

However, in the related art disclosed in PTL 1, there is a concern that the plunger will not be shifted to a set end point of reciprocating, for example, due to the wear of a component (wear of a bearing, sealing substance, or the like) of the pump such that it is not possible to achieve set pump volumetric efficiency or a set flow rate. That is, in a case where the plunger is not shifted to the set end point of reciprocating, the valve device is likely to enter into a closed state at an earlier timing than that during normal operation such that the amount of dialysate which is discharged is reduced by an amount in response to a changed state. Therefore, since the amount of dialysate which is supplied to the blood purification device is reduced, there is a problem in that blood purification efficiency (treatment efficiency) is lowered.

In order to solve the problem, it is considered that the related art disclosed in PTL 2 is used; however, in the related art, it is possible to monitor the shutout states (liquid-tight degrees of closed states) of the valve device on the suction port side and the valve device on the discharge port side but it is not possible to monitor whether or not set pump volumetric efficiency or a set flow rate is achieved. Such a problem is not limited to the duplex pump which supplies dialysate, but can also arise in a reciprocating pump in general which causes a liquid to be suctioned and discharging through the pump chambers by the reciprocating of a plunger (reciprocating device).

The present invention is made taking such problems into account and an object thereof is to provide a reciprocating pump which is able to monitor pump volumetric efficiency or a flow rate with ease and accuracy.

Solution to Problem

The invention is defined by the subject-matter of claim 1.

The invention of Claim 2 provides the reciprocating pump according to Claim 1, in which the detection device detects a period of time during which both the valve device on the suction port side and the valve device on the discharge port side are in the closed state in the predetermined cycle and the arithmetic device performs an arithmetic operation to obtain pump volumetric efficiency or a flow rate based on a comparison between the period of detection time detected by the detection device and a period of time during which both the valve device on the suction port side and the valve device on the discharge port side are in the closed state in the predetermined cycle during normal operation.

The invention of Claim 3 provides the reciprocating pump according to Claim 1, in which the detection device detects a period of time during which the valve device on the discharge side is in the opened state in the predetermined cycle and the arithmetic device performs an arithmetic operation to obtain pump volumetric efficiency or a flow rate based on a comparison between the period of detection time detected by the detection device and a period of time during which the valve device on the discharge side is in the opened state in the predetermined cycle during normal operation.

The invention of Claim 4 provides the reciprocating pump according to any one of Claims 1 to 3, in which the liquid contains a conductive liquid and the detection device includes a transmitting electrode unit and a receiving electrode unit which are disposed on the pump chamber and on the flow route on the suction port side or on the flow route on the discharge port side, respectively, which are positioned to interpose the valve device therebetween and a calculating device that is able to calculate a detection period of time during which the valve device is in the opened state or in the closed state based on a period of current application time between the transmitting electrode unit and the receiving electrode unit.

The invention of Claim 5 provides the reciprocating pump according to Claim 4, further including: a shutout determination device that is able to determine whether or not the valve device is in a good shutout state and to monitor the shutout state based on the current application between the transmitting electrode unit and the receiving electrode unit.

The invention of Claim 6 provides the reciprocating pump according to any one of Claims 1 to 5, in which the pump chamber is adapted to have a liquid supply-side pump chamber that communicates with a liquid supply-side suction port and a liquid supply-side discharge port and a liquid drainage-side pump chamber that communicates with a liquid drainage-side suction port and a liquid drainage-side discharge port, and has a configuration in which the valve device is provided for each of the liquid supply-side suction port, the liquid supply-side discharge port, the liquid drainage-side suction port, and the liquid drainage-side discharge port, the reciprocating device reciprocates in both the liquid supply-side pump chamber and the liquid drainage-side pump chamber, and thereby, liquids in the liquid supply-side pump chamber and the liquid drainage-side pump chamber are discharged from the liquid supply-side discharge port and the liquid drainage-side discharge port, respectively.

The invention of Claim 7 provides the reciprocating pump according to Claim 6, in which dialysate that is guided to a blood purification device flows in the liquid supply-side pump chamber, dialysate guided out from the blood purification device flows in the liquid drainage-side pump chamber, and the reciprocating of the reciprocating device enables dialysate to be supplied to the blood purification device and to be guided out from the blood purification device.

The invention of Claim 8 provides the reciprocating pump according to Claim 6 or 7, in which the arithmetic device obtains pump volumetric efficiency or a flow rate in the liquid supply-side pump chamber and in the liquid drainage-side pump chamber and is able to detect liquid leakage between the liquid supply-side discharge port and the liquid drainage-side suction port based on a comparison between pump volumetric efficiency or a flow rate in the liquid supply-side pump chamber and the pump volumetric efficiency or the flow rate in the liquid drainage-side pump chamber.

Advantageous Effects of Invention

According to the invention of Claim 1, the reciprocating pump includes the detection device that is able to detect the period of time during which the valve device is in the opened state or in the closed state in a predetermined cycle; and an arithmetic device that performs an arithmetic operation to obtain the pump volumetric efficiency or the flow rate based on a comparison between the period of detection time detected by the detection device and the period of time during which the valve device is in the opened state or in the closed state in the predetermined cycle during normal operation. Therefore, it is possible to monitor the pump volumetric efficiency or the flow rate with ease and accuracy.

According to the invention of Claim 2, the detection device detects the period of time during which both the valve device on the suction port side and the valve device on the discharge port side are in the closed state in the predetermined cycle and the arithmetic device performs an arithmetic operation to obtain the pump volumetric efficiency or the flow rate based on a comparison between the period of detection time detected by the detection device and the period of time during which both the valve device on the suction port side and the valve device on the discharge port side are in the closed state in the predetermined cycle during normal operation. Therefore, a relatively simple configuration makes it possible to monitor the pump volumetric efficiency or the flow rate with ease and accuracy.

According to the invention of Claim 3, the detection device detects the period of time during which the valve device on the discharge side is in the opened state in the predetermined cycle and the arithmetic device performs an arithmetic operation to obtain the pump volumetric efficiency or the flow rate based on a comparison between the period of detection time detected by the detection device and the period of time during which the valve device on the discharge side is in the opened state in the predetermined cycle during normal operation. Therefore, it is possible to simplify the detection by the detection device and the arithmetic operation by the arithmetic device and it is possible to monitor the pump volumetric efficiency or the flow rate with ease and accuracy.

According to the invention of Claim 4, the liquid contains the conductive liquid and the detection device includes the transmitting electrode unit and the receiving electrode unit which are disposed on the pump chamber and on the flow route on the suction port side or on the flow route on the discharge port side, respectively, which are positioned to interpose the valve device therebetween and a calculating device that is able to calculate a detection period of time during which the valve device is in the opened state or in the closed state based on a period of current application time between the transmitting electrode unit and the receiving electrode unit. Therefore, it is possible to monitor the pump volumetric efficiency or the flow rate with ease and reliability.

According to the invention of Claim 5, the shutout determination device that is able to determine whether or not the valve device is in a good shutout state and to monitor the shutout state based on the current application between the transmitting electrode unit and the receiving electrode unit is further provided. Therefore, it is possible to determine whether or not the shutout state is good in addition to the pump volumetric efficiency or the flow rate and it is possible to cause the shutout determination device to perform monitoring by diverting the transmitting electrode unit and the receiving electrode unit which configure the detection device.

According to the invention of Claim 6, the pump chamber is adapted to have the liquid supply-side pump chamber that communicates with the liquid supply-side suction port and the liquid supply-side discharge port, and the liquid drainage-side pump chamber that communicates with the liquid drainage-side suction port and the liquid drainage-side discharge port, and has a configuration in which the valve device is provided for each of the liquid supply-side suction port, the liquid supply-side discharge port, the liquid drainage-side suction port, and the liquid drainage-side discharge port, the reciprocating device reciprocates in both the liquid supply-side pump chamber and the liquid drainage-side pump chamber, and thereby, liquids in the liquid supply-side pump chamber and the liquid drainage-side pump chamber are discharged from the liquid supply-side discharge port and the liquid drainage-side discharge port, respectively. Therefore, it is possible to monitor the pump volumetric efficiency or the flow rate in one of or both the pump chambers of the liquid supply-side pump chamber and the liquid drainage-side pump chamber.

According to the invention of Claim 7, the dialysate that is guided to the blood purification device flows in the liquid supply-side pump chamber, the dialysate guided out from the blood purification device flows in the liquid drainage-side pump chamber, and the reciprocating of the reciprocating device enables the dialysate to be supplied to the blood purification device and to be guided out from the blood purification device. Therefore, it is possible to monitor that the amount of the dialysate which is supplied to the blood purification device is reduced and it is possible to prevent the pump volumetric efficiency from deteriorating.

According to the invention of Claim 8, the arithmetic device obtains the pump volumetric efficiency or the flow rate in the liquid supply-side pump chamber and in the liquid drainage-side pump chamber and is able to detect liquid leakage between the liquid supply-side discharge port and the liquid drainage-side suction port based on a comparison between the pump volumetric efficiency or the flow rate in the liquid supply-side pump chamber and the pump volumetric efficiency or the flow rate in the liquid drainage-side pump chamber. Therefore, it is possible to also detect the liquid leakage in addition to monitoring the pump volumetric efficiency or the flow rate in the liquid supply-side pump chamber and the liquid drainage-side pump chamber.

Brief Description of Drawings

[Fig. 1] Fig. 1 is a diagram schematically illustrating an entire dialysis apparatus to which a reciprocating pump according to a first embodiment of the present invention is applied.

[Fig. 2] Fig. 2 is a three-side view illustrating the external appearances of the reciprocating pump.

[Fig. 3] Fig. 3 is a sectional view taken along line III-III in Fig. 2.

[Fig. 4] Fig. 4 is a sectional view taken along line IV-IV in Fig. 2.

[Fig. 5] Fig. 5 is a sectional view illustrating an internal state in a process of the reciprocating of a reciprocating device in the reciprocating pump.

[Fig. 6] Fig. 6 is a sectional view illustrating an internal state in the process of the reciprocating of the reciprocating device in the reciprocating pump.

[Fig. 7] Fig. 7 is a sectional view illustrating an internal state in the process of the reciprocating of the reciprocating device in the reciprocating pump.

[Fig. 8] Fig. 8 is a sectional view illustrating an internal state in the process of the reciprocating of the reciprocating device in the reciprocating pump.

[Fig. 9] Fig. 9 is a block diagram illustrating a detection device in the reciprocating pump.

[Fig. 10] Fig. 10 is a graph illustrating an output signal of the detection device in the reciprocating pump, in which (a) is a graph illustrating an output signal by a voltage-current converting circuit, (b) is a graph illustrating an output signal by an absolute value amplifier circuit, and (c) is a graph illustrating an output signal by an integrating circuit.

[Fig. 11] Fig. 11 illustrates outputs of an integrating circuit on a suction side and of an integrating circuit on a discharge side, outputs of a voltage comparing circuit on the suction side and of a voltage comparing circuit on the discharge side, and an output of a logic circuit in the reciprocating pump during normal operation.

[Fig. 12] Fig. 12 illustrates outputs of the integrating circuit on the suction side and of the integrating circuit on the discharge side, outputs of the voltage comparing circuit on the suction side and of the voltage comparing circuit on the discharge side, and an output of the logic circuit in the reciprocating pump during abnormal operation.

[Fig. 13] Fig. 13 is a block diagram illustrating a detection device in a reciprocating pump according to a second embodiment of the present invention.

[Fig. 14] Fig. 14 illustrates outputs of a discharge-side integrating circuit and of a voltage comparing circuit during normal operation and outputs of the discharge-side integrating circuit and of the voltage comparing circuit.

[Fig. 15] Fig. 15 is a sectional view illustrating a reciprocating pump according to another embodiment of the present invention.

Description of Embodiments

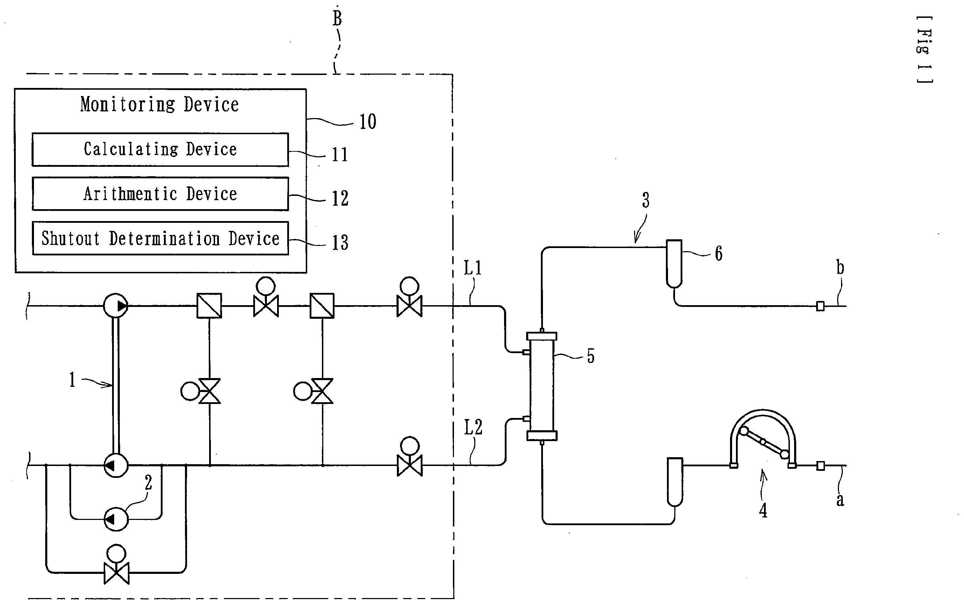

Hereinafter, embodiments of the present invention will be specifically described with reference to the drawings. A reciprocating pump according to the present embodiment is configured of a so-called duplex pump which is applied to a blood purification apparatus, which supplies a liquid from a liquid supply-side pump chamber to a blood purification device, and which causes the liquid drainage-side pump chamber to drain a dialysis effluent from the blood purification device. The blood purification apparatus to which the pump is applied is adapted of a hemodialysis apparatus for performing a hemodialysis treatment on a patient and, as illustrated in Fig. 1, is mainly configured to include a blood circuit 3 (an arterial blood circuit and a venous blood circuit) connected with a dialyzer 5 (blood purification device) and a dialysis device B which has a dialysate guiding-in line L1 and a dialysate discharging line L2.

Since the dialyzer 5 is used for purifying blood, the dialyzer 5 is connected with each of the arterial blood circuit and the venous blood circuit which configure the blood circuit 3, is connected to each of the dialysate guiding-in line L1 and the dialysate discharging line L2, and extracorporeally circulates blood collected through an arterial puncture needle a from a patient, though the blood circuit. After the blood is subjected to blood purification and ultrafiltration in the dialyzer 5, the blood returns into the patient through a venous puncture needle b. A reference sign 6 represents an air trap chamber.

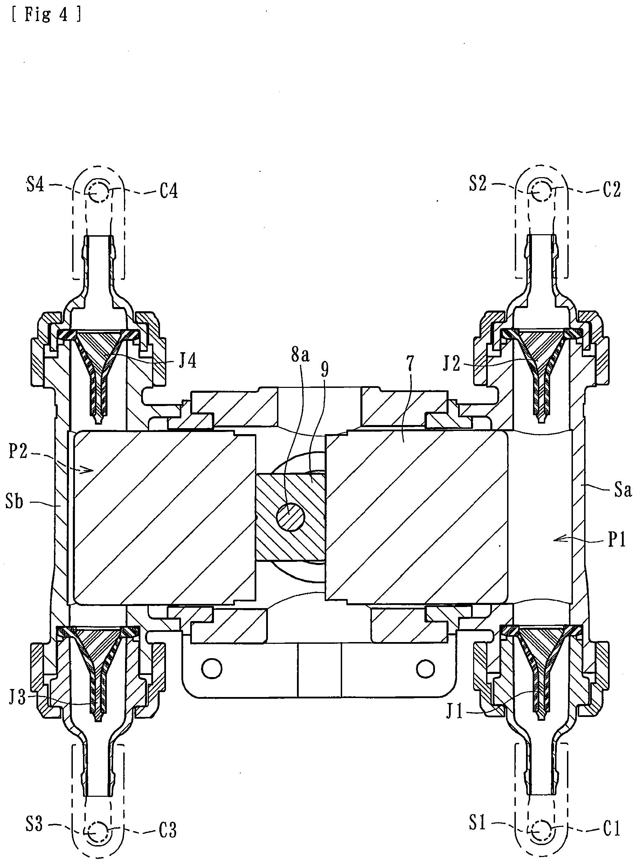

In addition, to the dialysate guiding-in line L1 and the dialysate discharging line L2, a duplex pump 1 (reciprocating pump) which supplies dialysate prepared to have a predetermined concentration to the dialyzer 5 and discharges dialysate (dialysis effluent) from the dialyzer 5 to the outside. Further, in the dialysis device B, a plurality of bypass lines or electromagnetic valves are disposed at arbitrary positions and an ultrafiltration pump 2 is connected to the bypass line which causes the liquid drainage-side pump chamber P2 (refer to Fig. 4) of the duplex pump 1 to be bypassed.

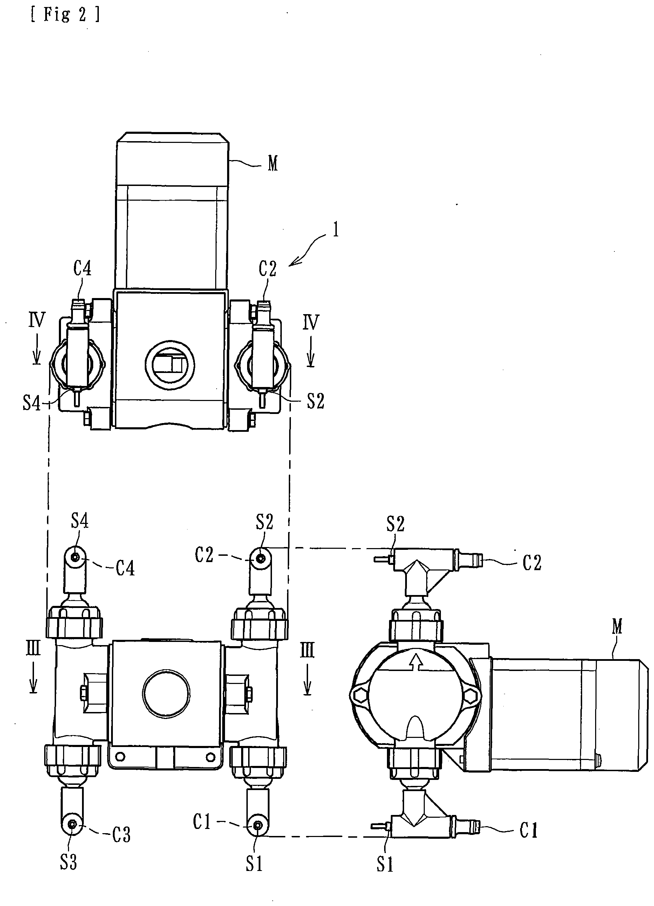

Here, as illustrated in Figs. 2 to 4, the duplex pump 1 according to the present embodiment is mainly configured to include a liquid supply-side pump chamber p1, the liquid drainage-side pump chamber p2, a liquid supply-side suction port C1 which enables the dialysate to be suctioned into the liquid supply-side pump chamber p1, a liquid supply-side discharge port C2 which enables the dialysate in the liquid supply-side pump chamber P1 to be discharged, a liquid drainage-side suction port C3 which enables the dialysate in the liquid drainage-side pump chamber P2 to be suctioned, a liquid drainage-side discharge port C4 which enables the dialysate in the liquid drainage-side pump chamber P2 to be discharged, a motor M as a drive device, a plunger 7 as a reciprocating device, check valves (J1 to J4) as valve devices, and a monitoring device 10 which has a calculating device 11, an arithmetic device 12 and a shutout determination device 13.

The liquid supply-side pump chamber P1 is formed of an accommodation space having a predetermined volume which enables the dialysate (liquid which is adapted of an electrolyte) suctioned from the liquid supply-side suction port C1 to flow and to be discharged from the liquid supply-side discharge port C2 due to the reciprocating of the plunger 7. Similarly, the liquid drainage-side pump chamber P2 is formed of an accommodation space having a predetermined volume which enables the dialysate suctioned from the liquid drainage-side suction port C3 to flow and to be discharged from the liquid drainage-side discharge port C4 due to the reciprocating of the plunger 7. In addition, each of the liquid supply-side suction port C1 and the liquid supply-side discharge port C2 is connected with piping which configures the dialysate guiding-in line L1 and each of the liquid drainage-side suction port C3 and the liquid drainage-side discharge port C4 is connected with piping which configures the dialysate discharging line L2.

The plunger 7 is able to reciprocate with respect to the liquid supply-side pump chamber P1 and the liquid drainage-side pump chamber P2 by the motor M, the reciprocating enables the dialysate to be suctioned into the liquid supply-side pump chamber P1 and the liquid drainage-side pump chamber P2 from the liquid supply-side suction port C1 and the liquid drainage-side suction port C3, respectively, and enables the dialysate to be discharged from the liquid supply-side discharge port C2 and the liquid drainage-side discharge port C4, respectively.

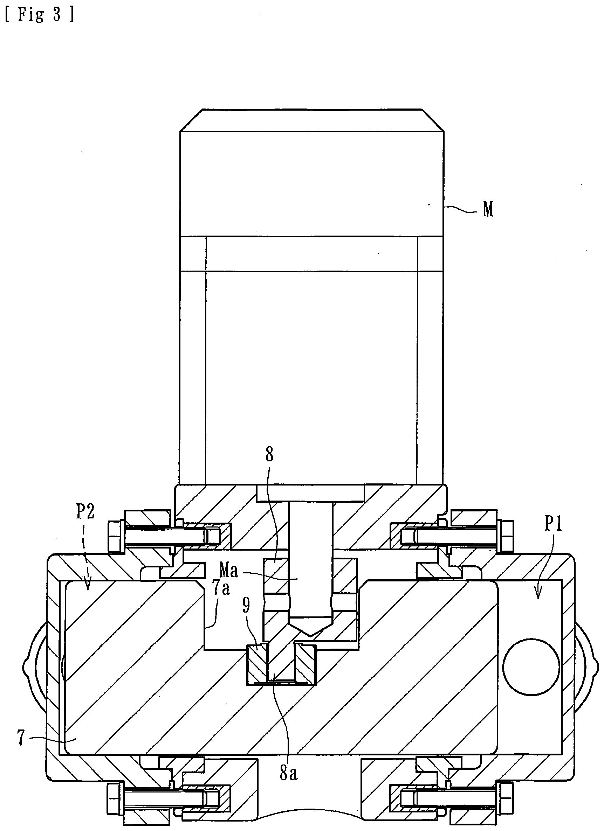

To be more specific, as illustrated in Figs. 3 and 4, a cam member 8 is attached to an output shaft Ma of the motor M and a protruding cam portion 8a is formed at a position (position shifted from the rotational center) different from an axial line of the output shaft Ma. The cam member 8 is positioned in a recessed portion 7a formed in the plunger 7 in a state in which the motor M is assembled thereto. In addition, when a block slider 9 is attached to the cam portion 8a and the motor M is driven, the block slider 9 presses the plunger 7 in a longitudinal direction such that the plunger 7 is caused to reciprocate in a rightward-leftward direction in Fig. 3.

The check valves (J1 and J2) are formed of a no-current applicable valve (valve formed of an insulating material) which has a function of maintaining the flow of the dialysate in one direction (upward in Fig. 4) such that back flow is prevented, and are configured to be disposed between the liquid supply-side pump chamber P1 and a flow route on the liquid supply-side suction port C1 side and a flow route on the liquid supply-side discharge port C2 side, respectively, are openable and closable in response to a change in a liquid pressure in the liquid supply-side pump chamber P1 due to the reciprocating of the plunger 7, allow the dialysate to flow in an opened state, and block the dialysate from flowing in a closed state.

Similarly, the check valves (J3 and J4) are formed of a no-current applicable valve (valve formed of an insulating material) which has a function of maintaining flow of the dialysate in one direction (upward in Fig. 4) such that back flow is prevented, and are configured to be disposed between the liquid drainage-side pump chamber P2 and a flow route on the liquid drainage-side suction port C3 side and a flow route on the liquid drainage-side discharge port C4 side, respectively, are openable and closable in response to a change in a liquid pressure in the liquid drainage-side pump chamber P2 due to the reciprocating of the plunger 7, allow the dialysate to flow in the opened state, and block the dialysate from flowing in the closed state.

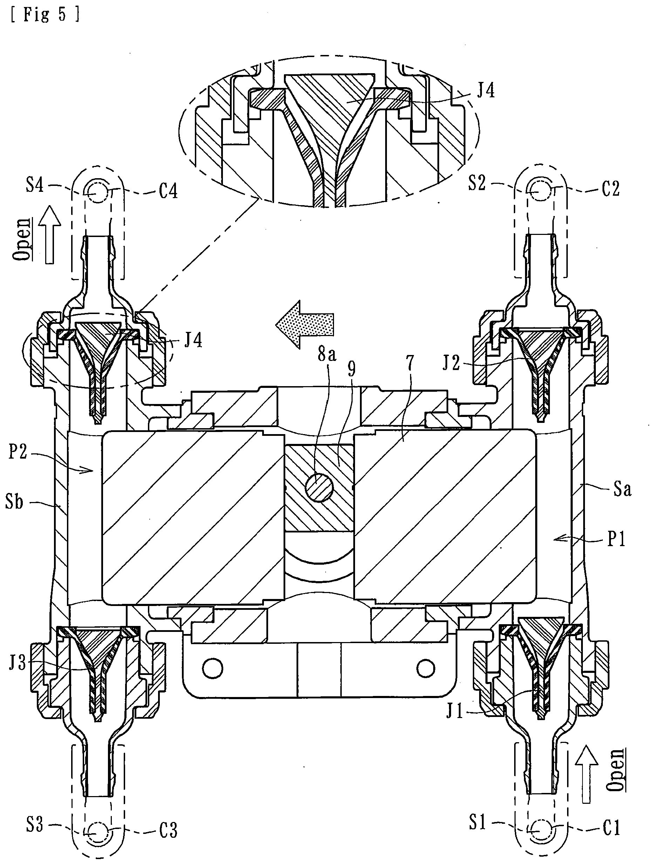

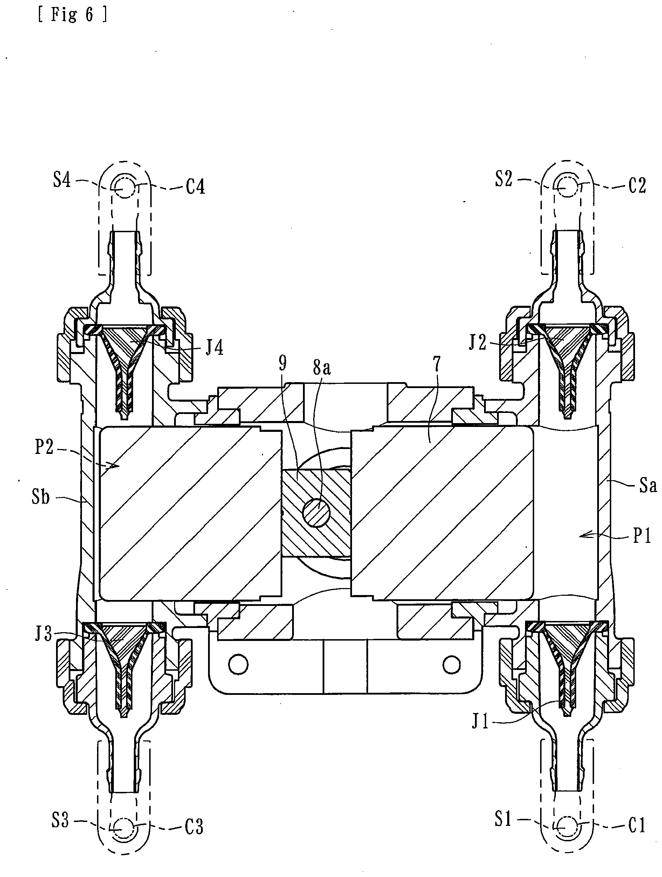

Thus, in a process in which the plunger 7 is shifted to the left side in the drawing to reach an end portion (shifted end) on the liquid drainage-side pump chamber P2 side, when the motor M is driven, as illustrated in Fig. 5, the check valve J1 on the liquid supply-side suction port C1 side and the check valve J4 on the liquid drainage-side discharge port C4 side enter into the opened state and the check valve J2 on the liquid supply-side discharge port C2 side and the check valve J3 on the liquid drainage-side suction port C3 side enter into the closed state. Then, when the plunger 7 reaches the left end (shifted end) in the drawing, as illustrated in Fig. 6, all of the check valves (J1 to J4) enter into the closed state.

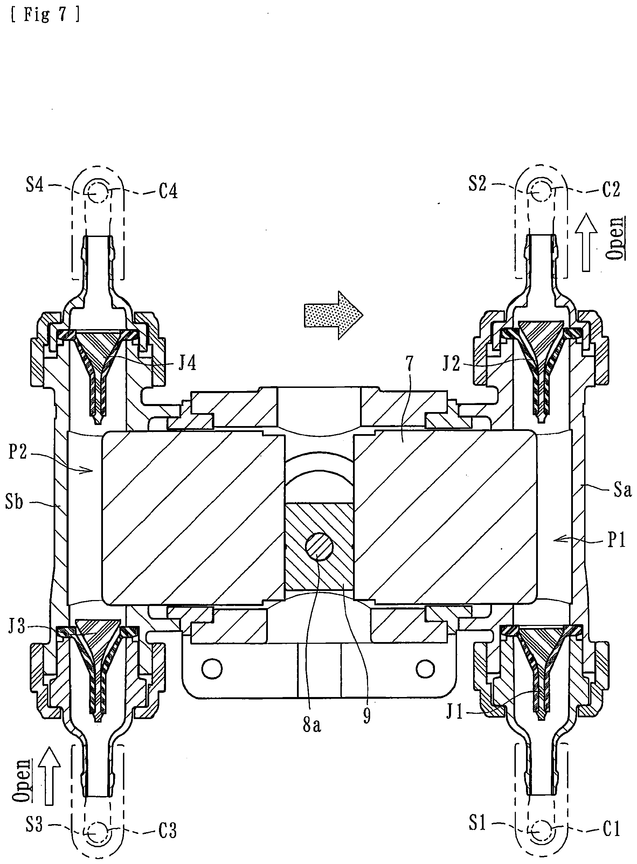

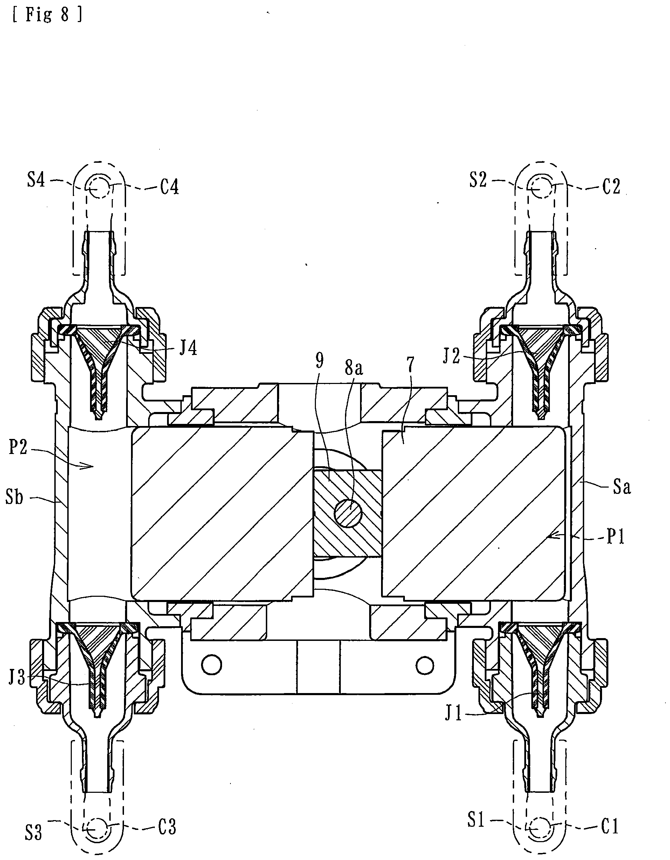

Then, in a process in which the plunger 7 is shifted to the right side in the drawing to reach an end portion (shifted end) on the liquid supply-side pump chamber P1 side, as illustrated in Fig. 7, the check valve J2 on the liquid supply-side discharge port C2 side and the check valve J3 on the liquid drainage-side suction port C3 side enter into the opened state and the check valve J1 on the liquid supply-side suction port C1 side and the check valve J4 on the liquid drainage-side discharge port C4 side enter into the closed state. Then, when the plunger 7 reaches the right end (shifted end) in the drawing, as illustrated in Fig. 8, all of the check valves (J1 to J4) enter into the closed state. Thereafter, the same opening and closing operation of the check valves (J1 to J4) are performed due to the reciprocating of the plunger 7.

The detection device can detect a period of time during which the check valves (J1 to J4) are in the opened state or in the closed state in a predetermined cycle (according to the present embodiment, one logical cycle when a shot is performed or an opening/closing cycle of the check valve). According to the present embodiment, the detection device is configured to include a transmitting electrode unit Sa and receiving electrode units (S1 and S2) which are disposed on the liquid supply-side pump chamber P1 and on the flow route on the liquid supply-side suction port C1 or on the flow route on the liquid supply-side discharge port C2 side, respectively, which are positioned to interpose the check valves (J1 and J2) therebetween, a transmitting electrode unit Sb and receiving electrode units (S3 and S4) which are disposed on the liquid drainage-side pump chamber P2 and on the flow route on the liquid drainage-side suction port C3 side or on the flow route on the liquid drainage-side discharge port C4 side, respectively, which are positioned to interpose the check valves (J3 and J4) therebetween. That is, the detection device according to the present embodiment is configured to include the electrode portions (S1 to S4 and Sa and Sb) and a calculating device 11.

Specifically, the transmitting electrode unit Sa is formed of a conductive member which configures a part of the liquid supply-side pump chamber P1 and is formed of a conductive electrode member to which the receiving electrode unit S1 is attached to face the flow route (flow route closer to the liquid supply-side suction port C1 side than a provided position of the check valve J1) in the vicinity of the liquid supply-side suction port C1 and the receiving electrode unit S2 is attached to face the flow route (closer to the liquid supply-side discharge port C2 side than a provided position of the check valve J2) in the vicinity of the liquid supply-side discharge port C2.

In addition, the transmitting electrode unit Sb is formed of a conductive member which configures a part of the liquid drainage-side pump chamber P2 and is formed of a conductive electrode member to which the receiving electrode unit S3 is attached facing the flow route (flow route closer to the liquid drainage-side suction port C3 side than a provided position of the check valve J3) in the vicinity of the liquid drainage-side suction port C3 and the receiving electrode unit S4 is attached to face the flow route (closer to the liquid drainage-side discharge port C4 side than a provided position of the check valve J4) in the vicinity of the liquid drainage-side discharge port C4.

In the process in which the plunger 7 reciprocates, when the check valves J1 and J4 enter into the opened state and the check valves J2 and J3 enter into the closed state, the current is applied to the transmitting electrode unit Sa and the receiving electrode unit S1, and the transmitting electrode unit Sb and the receiving electrode unit S4 and no current is applied to the transmitting electrode unit Sa and the receiving electrode unit S2, or to the transmitting electrode unit Sb and the receiving electrode unit S3 because the dialysate contains a conductive liquid and the check valves J1, J2, J3, and J4 are formed of a no-current applicable material. Further, in the process in which the plunger 7 reciprocates, when the check valves J2 and J3 enter into the opened state and the check valves J1 and J4 enter into the closed state, no current is applied to the transmitting electrode unit Sa and the receiving electrode unit S1, or the transmitting electrode unit Sb and the receiving electrode unit S4 and the current is applied to the transmitting electrode unit Sa and the receiving electrode unit S2, and to the transmitting electrode unit Sb and the receiving electrode unit S3 because the dialysate contains a conductive liquid and the check valves J1, J2, J3, and J4 are formed of a no-current applicable material.

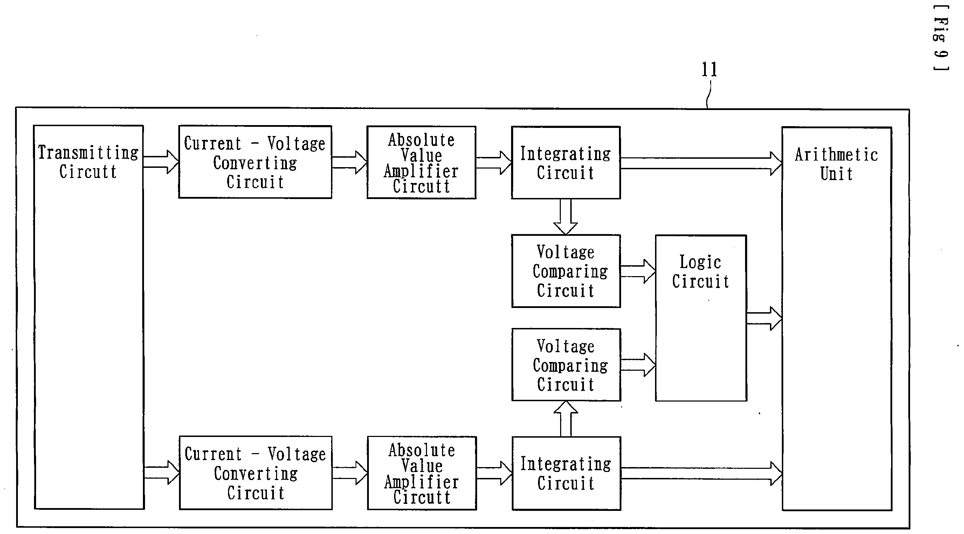

The calculating device 11 is able to calculate a period of detection time during which the check valves (J1 to J4) are in the opened state or in the closed state, based on a period of current application time between the transmitting electrode units (Sa and sb) and the receiving electrode units (S1 to S4). Therefore, according to the present embodiment, as illustrated in Fig. 9, the calculating device 11 is configured to include a single transmitting circuit, a pair of current-voltage converting circuits, a pair of absolute value amplifier circuits, a pair of integrating circuits, a pair of voltage comparing circuits, a single logic circuit, and an arithmetic unit. In the same drawing, the calculating device includes two of a suction-side calculating device on the liquid supply-side suction port C1 side and an discharge-side calculating device on the liquid supply-side discharge port C2 side on the liquid supply-side pump chamber P1 side, which are depicted in the drawing; similarly, in the present embodiment, the calculating device 11 has two of a suction-side calculating device on the liquid drainage-side suction port C3 side and an discharge-side calculating device on the liquid drainage-side discharge port C4 side on the liquid drainage-side pump chamber P2. Hereinafter, the flow routes on the liquid supply-side suction port C1 side and the liquid supply-side discharge port C2 side on the liquid supply-side pump chamber P1 side will be described; and the same is true of the liquid drainage-side pump chamber P2 side.

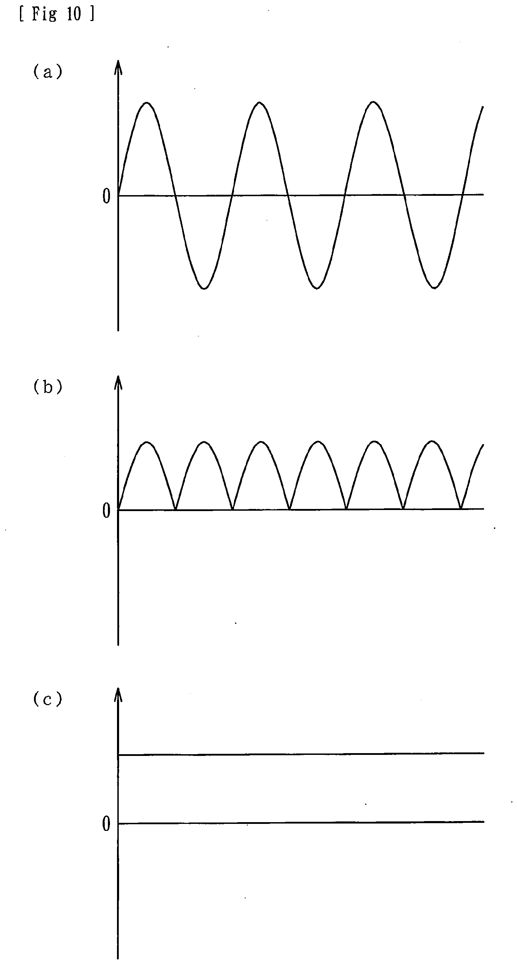

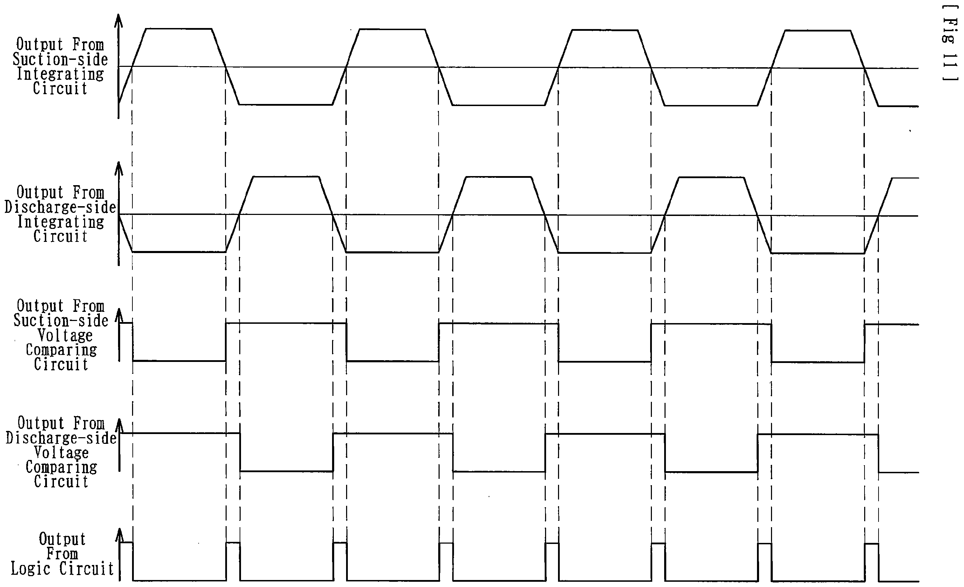

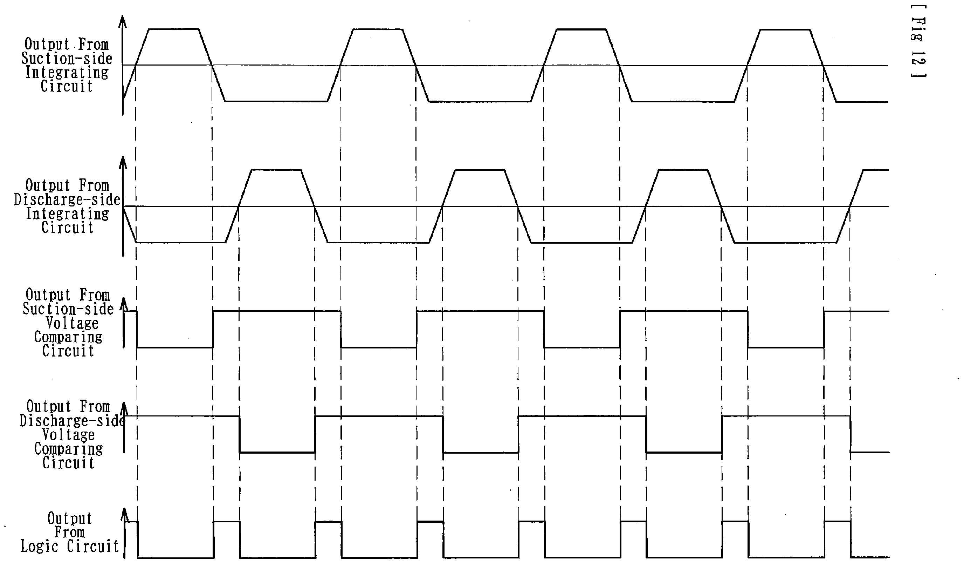

For example, as illustrated in Fig. 10, an electrical signal detected by the transmitting circuit is converted into a waveform (refer to the same drawing (a)) of a voltage in the current-voltage converting circuit, a waveform as illustrated in (b) in the same drawing is formed in the absolute value amplifier circuit, and then, a waveform as illustrated in (c) in the same drawing is formed in the integrating circuit. Thus, as illustrated in Figs. 11 and 12, the electrical signal detected in the transmitting circuit is subjected to a process by the integrating circuit of the suction-side calculating device and the integrating circuit of the discharge-side calculating device, then, a waveforms as illustrated in Figs. 11 and 12 (upper two waveforms in the same drawing) are output and the output waveforms are subjected to a process by the voltage comparing circuit of the suction-side calculating device and by the voltage comparing circuit of the discharge-side calculating device such that two-value signals (signals formed of two high and low levels) are formed (refer to the third and fourth waveforms from the top in the same drawing). In the same drawing, the high and low levels of the signal are inverted into the two-value output.

The logic circuit compares the output by the voltage comparing circuit of the suction-side calculating device with the output by the voltage comparing circuit of the discharge-side calculating device such that a waveform showing a timing at which both outputs have the high level (that is, both the check valve J1 on the liquid supply-side suction port C1 side and the check valve J2 on the liquid supply-side discharge port C2 side are in the closed state) is output (refer to the lowermost waveform in the same drawing). Thus, the arithmetic unit calculates a period of time (referred to as a detection time) during which the check valve J1 and the check valve J2 are in the closed state in a predetermined cycle (in this case, an opening/closing cycle of the check valve), based on the waveform output from the logic circuit.

The arithmetic device 12 performs an arithmetic operation to obtain pump volumetric efficiency (efficiency of one shot when the liquid is discharged in the reciprocating pump (metering pump)) or a flow rate by a predetermined arithmetic operation based on a comparison between the period of detection time detected by the detection device and a period of time (period of time as a measured value or a theoretical period of time during normal operation) during which the check valves (J1 and J2) are in the closed state in the predetermined cycle during normal operation. For example, in a case where the arithmetic device 12 performs the arithmetic operation to obtain the pump volumetric efficiency, it is possible to use an arithmetic equation as follows. An arithmetic equation 1 is used for obtaining the pump volumetric efficiency and an arithmetic equation 2 is used for obtaining the flow rate.

(Arithmetic Equation 1)

$$\text{Pump Volumetric Efficiency (\%)} = (\Delta T - TLL) / (\Delta T - \text{Initial } TLL)$$

Here, $\Delta T$ represents a period of logical one-cycle time or an opening/closing cycle of the check valve, TLL represents a period of detection time (period of time during which both the check valve on the suction side and the check valve on the discharge side are in the closed state in one cycle), and initial TLL represents a period of time during which both the check valve on the suction side and the check valve on the discharge side are in the closed state in one cycle during normal operation.

(Arithmetic Equation 2)

$$\text{Flow Rate (mL/min)} = (\Delta T - TLL) / (\Delta T - \text{Initial } TLL) \times QD$$

Here, QD (mL/min)=one shot of a reciprocating pump (metering pump) x 60 x 1/$\Delta T$(mL/min), and the other parameters are the same as arithmetic equation 1.

Meanwhile, the shutout determination device 13 is able to determine whether or not the shutout states of the check valves (J1 to J4) are good based on the current application between the transmitting electrode units (Sa and Sb) and the receiving electrode units (S1 to S4). That is, the current application between the transmitting electrode units (Sa and Sb) and the receiving electrode units (S1 to S4) is performed at a predetermined timing in response to the reciprocating of the plunger 7. When the shutout of check valves (J1 to J4) is not good, a period of no-current applicable time between the transmitting electrode units (Sa and Sb) and the receiving electrode units (S1 to S4) in which the check valves (J1 to J4) are formed. Therefore, the shutout determination device 13 determines the change and thereby it is possible to monitor the shutout state of the check valves (J1 to J4).

In addition, the arithmetic device 12 obtains pump volumetric efficiency or a flow rate of the liquid supply-side pump chamber P1 and the liquid drainage-side pump chamber P2 and may be able to detect liquid leakage from the flow route between the liquid supply-side discharge port C2 and the liquid drainage-side suction port C3 through the dialyzer 5, based on a comparison between the pump volumetric efficiency or the flow rate of the liquid supply-side pump chamber P1 and the pump volumetric efficiency or the flow rate of the liquid drainage-side pump chamber P2. In this case, it is possible to detect the liquid leakage from the flow route between the liquid supply-side discharge port C2 and the liquid drainage-side suction port C3 through the dialyzer 5, in addition to monitoring the pump volumetric efficiency or the flow rate of the liquid supply-side pump chamber P1 and the liquid drainage-side pump chamber P2.

Hereinafter, a second embodiment according to the present invention will be described. Similar to the first embodiment, the reciprocating pump according to the present embodiment is formed of a so-called duplex pump which is applied to a blood purification apparatus, which supplies a liquid from a liquid supply-side pump chamber to a blood purification device, and which causes the liquid drainage-side pump chamber to discharge dialysis effluent from the blood purification device. Since a main configuration in the blood purification apparatus and the duplex pump 1 to which the reciprocating pump is applied (components other than the calculating device 11 and the arithmetic device 12) is the same as that in the first embodiment, the description thereof is omitted.

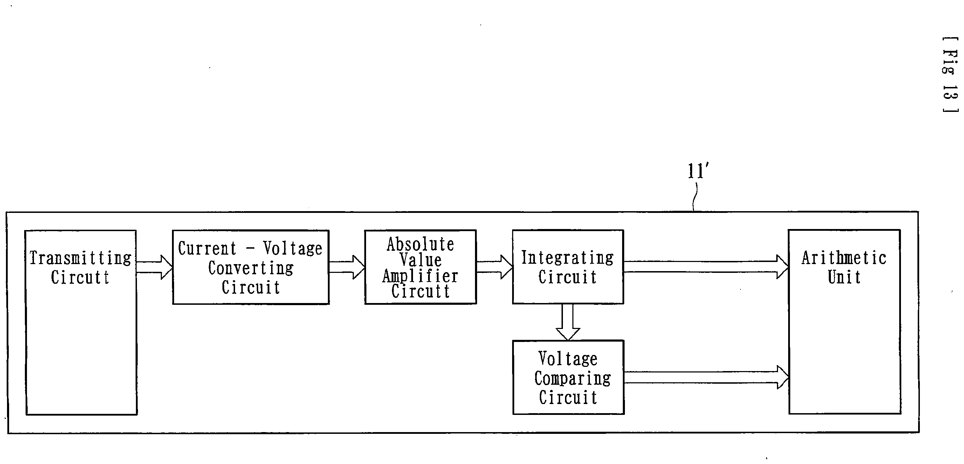

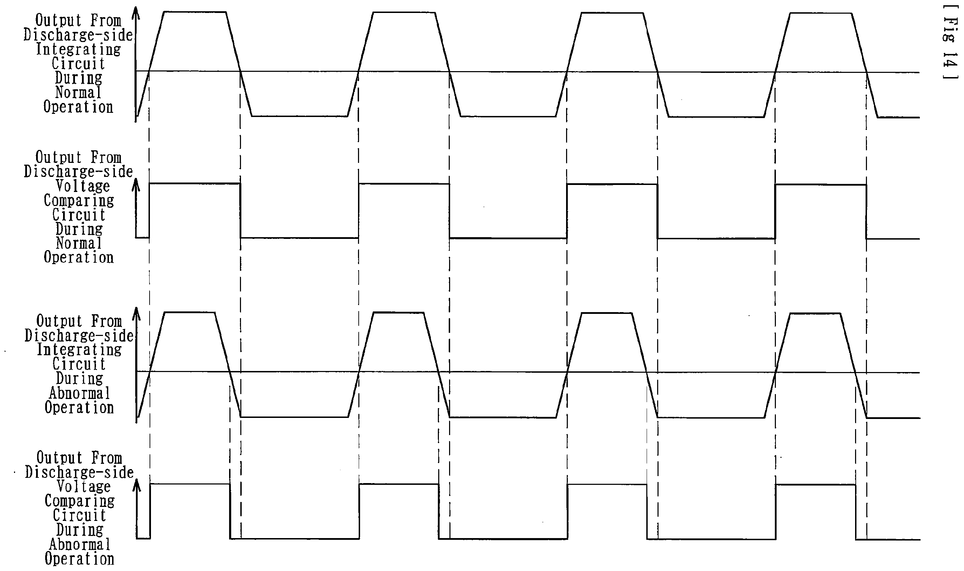

As illustrated in Fig. 13, the calculating device 11' according to the present embodiment is configured to include a single transmitting circuit, a single current-voltage converting circuit, a single absolute value amplifier circuit, a single integrating circuit, a single voltage comparing circuit, and an arithmetic unit. In the same drawing, the calculating device includes only one circuit on the liquid supply-side discharge port C2 side in the liquid supply-side pump chamber P1 side, which are depicted in the drawing; and similarly, in the present embodiment, the calculating device also include the circuit on the liquid drainage-side discharge port C4 in the liquid drainage-side pump chamber P2 side. Hereinafter, the circuit on the liquid supply-side discharge port C2 side in the liquid supply-side pump chamber P1 side will be described and the same is true of the liquid drainage-side pump chamber P2 side. During normal operation, the electrical signal detected in the transmitting circuit is processed in the integrating circuit, a waveform as illustrated on the top in Fig. 14 is output, and the output waveform is changed in the voltage comparing circuit to have two values (processed to form a signal having two high and low signals), the second waveform output from the top as illustrated in the same drawing is obtained.

However, the electrical signal detected in the transmitting circuit is processed in the integrating circuit during usage process of the reciprocating pump, the second waveform from below as illustrated in Fig. 14 is output, the output waveform is processed in the voltage comparing circuit to have two values (processed to a signal formed of two high and low levels), and the lowermost output as illustrated in the same drawing is achieved. The arithmetic device can perform an arithmetic operation to obtain the pump volumetric efficiency (efficiency of one shot when the liquid is discharged in the reciprocating pump (metering pump)) or a flow rate based on the output obtained during normal operation and the output obtained during abnormal operation. Hereinafter, the arithmetic equations of the present embodiment (an arithmetic equation 3 is used for obtaining the pump volumetric efficiency and an arithmetic equation 4 is used for obtaining the flow rate) will be described.

(Arithmetic Equation 3)

Pump Volumetric Efficiency (%)=(TH)/(Initial TH)

Here, TH represents a period of detection time (period of time during which the check valve on the discharge side is in the opened state in one cycle), and initial TH represents a period of time during which the check valve on the discharge side is in the opened state in one cycle during normal operation.

(Arithmetic Equation 4)

Flow Rate (mL/min)=TH/Initial TH x QD

Here, QD (mL/min)= one shot of a reciprocating pump (metering pump) x 60 x 1/ΔT(mL/min), and the other parameters are the same as arithmetic equation 3.

According to the first embodiment and the second embodiment, the detection device (transmitting electrode units Sa and Sb and receiving electrode units (S1 to S4)) which can detect a period of time during which the check valves (J1 to J4) are in the opened state or in the closed state in the predetermined cycle and the arithmetic device 12 which performs the arithmetic operation to obtain the pump volumetric efficiency and the flow rate based on a comparison between the detection time detected in the detection device and the period of time during which the check valves (J1 to J4) are in the opened state or in the closed state in the predetermined cycle during normal operation. Therefore, it is possible to monitor the pump volumetric efficiency or the flow rate with ease and accuracy.

In addition, the liquid contains the conductive liquid (that is, dialysate) and the detection device includes the transmitting electrode units (Sa and Sb) and the receiving electrode units (S1 to S4) which are disposed on the pump chambers (liquid supply-side pump chamber P1 and the liquid drainage-side pump chamber P2) and on the flow route on the suction ports (C1 and C3) side or on the flow route on the discharge port (C2 and C4) side, respectively, which are positioned to interpose the check valves (J1 to J4) therebetween and a calculating device (11 or 11') that is able to calculate a detection period of time during which the check valves (J1 to J4) are in the opened state or in the closed state based on a period of current application time between the transmitting electrode units (Sa and Sb) and the receiving electrode units (S1 to S4). Therefore, it is possible to monitor the pump volumetric efficiency or the flow rate with ease and reliability.

The shutout determination device 13 that is able to determine whether or not the check valves (J1 to J4) are in a good shutout state and to monitor the shutout state based on the current application between the transmitting electrode units (Sa and Sb) and the receiving electrode units (S1 to S4) is provided. Therefore, it is possible to determine whether or not the shutout state is good in addition to the pump volumetric efficiency or the flow rate and it is possible to cause the shutout determination device 13 to perform monitoring by diverting the transmitting electrode units (Sa and Sb) and the receiving electrode units (S1 to S4) which configure the detection device.

Further, the pump chamber is adapted to have the liquid supply-side pump chamber P1 that communicates with the liquid supply-side suction port C1 and the liquid supply-side discharge port C2 and the liquid drainage-side pump chamber P2 that communicates with the liquid drainage-side suction port C3 and the liquid drainage-side discharge port C4, and has a configuration in which the check valves (J1 to J4) are provided for each of the liquid supply-side suction port C1, the liquid supply-side discharge port C2, the liquid drainage-side suction port C3, and the liquid drainage-side discharge port C4, the plunger 7 (reciprocating device) reciprocates in both the liquid supply-side pump chamber P1 and the liquid drainage-side pump chamber P2, and thereby, dialysate (liquids) in the liquid supply-side pump chamber P1 and the liquid drainage-side pump chamber P2 is discharged from the liquid supply-side discharge port C2 and the liquid drainage-side discharge port C4, respectively. Therefore, it is possible to monitor the pump volumetric efficiency or the flow rate in one of or both the pump chambers of the liquid supply-side pump chamber P1 and the liquid drainage-side pump chamber P2.

The dialysate that is guided to the dialyzer 5 (blood purification device) flows in the liquid supply-side pump chamber P1, the dialysate guided out from the dialyzer 5 flows in the liquid drainage-side pump chamber P2, and the reciprocating of the plunger 7 (reciprocating device) enables the dialysate to be supplied to the dialyzer 5 and to be guided out from the dialyzer 5. Therefore, it is possible to monitor that the amount of the dialysate which is supplied to the dialyzer 5 is reduced and it is possible to prevent the pump volumetric efficiency from deteriorating.

Specifically, according to the first embodiment, the detection device detects a period of time during which both the check valve J1 on the suction port side and the check valve J2 on the discharge port side are in the closed state in the predetermined cycle and the arithmetic device 12 performs an arithmetic operation to obtain the pump volumetric efficiency or the flow rate based on a comparison between the period of detection time detected by the detection device and the period of time during which both the check valve J1 on the suction port side and the check valve J2 on the discharge port side are in the closed state in the predetermined cycle during normal operation. Therefore, a relatively simple configuration makes it possible to monitor the pump volumetric efficiency or the flow rate with ease and accuracy.

In addition, according to the second embodiment, the detection device detects the period of time during which the check valve J2 on the discharge port side is in the opened state in the predetermined cycle and the arithmetic device 12 performs an arithmetic operation to obtain the pump volumetric efficiency or the flow rate based on a comparison between the period of detection time detected by the detection device and the period of time during which the check valve J2 on the discharge port side is in the opened state in the predetermined cycle during normal operation. Therefore, it is possible to simplify the detection by the detection device and the arithmetic operation by the arithmetic device and it is possible to monitor the pump volumetric efficiency or the flow rate with ease and accuracy.

As above, the present embodiment is described; however, the present invention is not limited thereto and, for example, instead of the check valves (J1 to J4), another shape of a valve device may be used or the shutout determination device 13 may not be provided. The apparatus to which the valve device is applied is not limited to the blood purification apparatus (hemodialysis apparatus) as in the present embodiment and, in this case, the liquid which flows in the pump chamber is not limited to the dialysate.

In addition, as long as the reciprocating pump includes the detection device that is able to detect a period of time during which the valve device is in the opened state or in the closed state in a predetermined cycle; and an arithmetic device that performs an arithmetic operation to obtain the pump volumetric efficiency or the flow rate based on the comparison between the period of detection time detected by the detection device and a period of time during which the valve device is in the opened state or in the closed state in the predetermined cycle during normal operation, the invention may be applied to another shape of a pump.

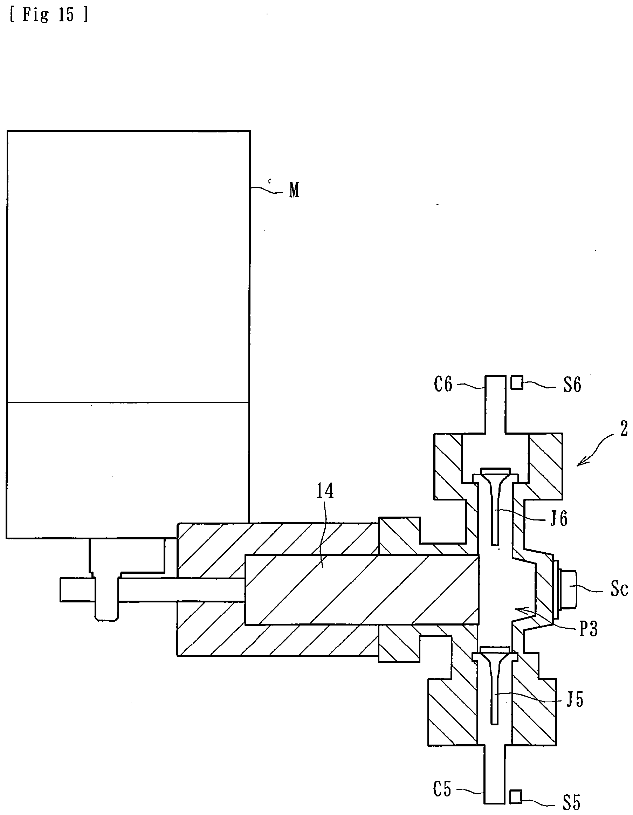

For example, as illustrated in Fig. 15, the present invention may be applied to the ultrafiltration pump 2 that includes a single pump chamber P3 which is able to flow a liquid, a suction port C5 which enables a liquid to be suctioned into the pump chamber P3, an discharge port C6 which enable a liquid in the pump chamber P3 to be discharged, a plunger 14 (reciprocating device) which is capable of reciprocating in the pump chamber P3 by the drive device such as the motor M and enables a liquid to be suctioned into the pump chamber P3 from the suction port C5 and enables the liquid to be discharged from the discharge port C6 due to the reciprocating, and check valves (J5 and J6) (valve devices) which are disposed between the pump chamber P3 and a flow route on the suction port C5 side and a flow route on the discharge port C6 side, are openable and closable in response to a change in a liquid pressure in the pump chamber P3, which is produced due to the reciprocating of the plunger 14, allow a liquid to flow in the opened state, and block a liquid from flowing in the closed state.

In this case, it is preferable that the calculating device 11 that configures the detection device calculates a period of detection time during which the check valves (J5 and J6) are in the opened state or in the closed state based on a period of current application time between a transmitting electrode unit Sc and receiving electrode units (S5 and S6) and the arithmetic device 12 performs an arithmetic operation to obtain pump volumetric efficiency or a flow rate based on the period of detection time detected by the detection device and the period of time during which the check valves (J5 and J6) are in the opened state or in the closed state in the predetermined cycle during normal operation.

Industrial Applicability

As long as a reciprocating pump includes a detection device that is able to detect a period of time during which the valve device is in the opened state or in the closed state in a predetermined cycle; and an arithmetic device that performs an arithmetic operation to obtain pump volumetric efficiency or a flow rate based on a comparison between the period of detection time detected by the detection device and a period of time during which the valve device is in the opened state or in the closed state in the predetermined cycle during normal operation, the invention may be applied to another pump having a different external appearance or a pump to which another function is added.

Reference Signs List

| 1 | duplex pump (reciprocating pump) |
|---|---|
| 2 | ultrafiltration pump (reciprocating pump) |
| 3 | blood circuit |
| 4 | blood pump |
| 5 | dialyzer (blood purification device) |
| 6 | air trap chamber |
| 7 | plunger (reciprocating device) |
| 8 | cam member |
| 9 | block slider |
| 10 | monitoring device |
| 11 | calculating device (detection device) |
| 12 | arithmetic device |
| 13 | shutout determination device |
| 14 | plunger (reciprocating device) |
| J1 to J6 | check valve (valve device) |
| P1 | liquid supply-side pump chamber |
| P2 | liquid drainage-side pump chamber |
| C1 | liquid supply-side suction port |
| C2 | liquid supply-side discharge port |
| C3 | liquid drainage-side suction port |
| C4 | liquid drainage-side discharge port |
| Sa, Sb | transmitting electrode unit (detection device) |
| S1 to S4 | receiving electrode unit (detection device) |

**Claims**

**1.** A reciprocating pump (1) comprising:

a pump chamber (P1) which is able to flow a liquid;
a suction port (C1) which enables a liquid to be suctioned into the pump chamber (P1);
a discharge port (C2) which enables a liquid in the pump chamber (P1) to be discharged;
a reciprocating device (7) which is adapted to reciprocate n the pump chamber (P1) by a drive device and enables a liquid to be suctioned into the pump chamber (P1) from the suction port (C1) and enables the liquid to be discharged from the discharge port (C2) due to the reciprocating;
valve devices (J1, J2) which are disposed between the pump chamber (P1) and a flow route on a suction port side and a flow route on a discharge port side,
are openable and closable in response to a change in a liquid pressure in the pump chamber (P1), which is produced depending on the reciprocating of the reciprocating device (7), to allow the liquid to flow in an opened state, and block the liquid from flowing in a closed state;
**characterized in**
a detection device that is able to detect a period of time during which the valve devices (J1,J2) are in the opened state or in the closed state in a predetermined cycle; and
an arithmetic device (12) that performs an arithmetic operation to obtain pump volumetric efficiency or a flow rate based on a comparison between the period of detection time detected by the detection device and a period of time during which the valve devices (J1 ,J2) are in the opened state or in the closed state in the predetermined cycle during a wearless operational state of the reciprocating pump.

2. The reciprocating pump according to Claim 1,
   **characterized in that**
   the detection device detects a period of time during which both the valve device (J1) on the suction port side and the valve device (J2) on the discharge port side are in the closed state in the predetermined cycle and the arithmetic device (12) performs an arithmetic operation to obtain pump volumetric efficiency or a flow rate based on a comparison between the period of detection time detected by the detection device and a period of time during which both the valve device (J1) on the suction port side and the valve device (J2) on the discharge port side are in the closed state in the predetermined cycle during a wearless operational state of the reciprocating pump.

3. The reciprocating pump according to Claim 1,
   **characterized in that**
   the detection device detects a period of time during which the valve device (J2) on the discharge side is in the opened state in the predetermined cycle and the arithmetic device (12) performs an arithmetic operation to obtain pump volumetric efficiency or a flow rate based on a comparison between the period of detection time detected by the detection device and a period of time during which the valve device (J2) on the discharge side is in the opened state in the predetermined cycle during a wearless operational state of the reciprocating pump.

4. The reciprocating pump according to any one of Claims 1 to 3,
   **characterized in that**
   the liquid contains a conductive liquid and the detection device includes a transmitting electrode unit and a receiving electrode unit which are disposed on the pump chamber and on the flow route on the suction port side or on the flow route on the discharge port side, respectively, which are positioned to interpose the valve device therebetween and a calculating device that is able to calculate a detection period of time during which the valve device is in the opened state or in the closed state based on a period of current application time between the transmitting electrode unit and the receiving electrode unit (S1,S2).

5. The reciprocating pump according to Claim 4,
   **characterized in that** it further comprises:
   a shutout determination device (13) that is able to determine whether or not the valve device (J1, J2) is in a good shutout state and to monitor the shutout state based on the current application between the transmitting electrode unit (Sa) and the receiving electrode unit (S1, S2).

6. The reciprocating pump according to any one of Claims 1 to 5,
   **characterized in that**
   the pump chamber (P1) is adapted to have a liquid supply-side pump chamber (P1) that communicates with a liquid supply-side suction port (C1) and a liquid supply-side discharge port (C2) and a liquid drainage-side pump chamber (P2) that communicates with a liquid drainage-side suction port (C3) and a liquid drainage-side discharge port (C4), and has a configuration in which the valve device (J1, J2, J3, J4) is provided for each of the liquid supply-side suction port, the liquid supply-side discharge port, the liquid drainage-side suction port, and the liquid drainage-side discharge port, the reciprocating device (7) is adapted to reciprocate in both the liquid supply-side pump chamber (P1) and the liquid drainage-side pump chamber (P2), and wherein, liquids in the liquid supply-side pump chamber (P1) and the liquid drainage-side pump chamber (P2) are discharged from the liquid supply-side discharge port (C2) and the liquid drainage-side discharge port (C4), respectively.

7. The reciprocating pump according to Claim 6,
   **characterized in that**
   dialysate that is guided to a blood purification device (5) flows in the liquid supply-side pump chamber (P1), dialysate guided out from the blood purification device (5) flows in the liquid drainage-side pump chamber (P2), and the reciprocating of the reciprocating device (7) enables dialysate to be supplied to the blood purification device (5) and to be guided out from the blood purification device (5).

8. The reciprocating pump according to Claim 6 or 7,
   **characterized in that**
   the arithmetic device (12) obtains pump volumetric efficiency or a flow rate in the liquid supply-side pump chamber (P1) and in the liquid drainage-side pump chamber (P2) and is able to detect liquid leakage between the liquid supply-side discharge port (C2) and the liquid drainage-side suction port (C3) based on a comparison between pump volumetric efficiency or a flow rate in the liquid supply-side pump chamber (P1) and the pump volumetric efficiency or the flow rate in the liquid drainage-side pump chamber (P2).

**Patentansprüche**

1. Kolbenpumpe (1) mit:

   einer Pumpenkammer (P1), welche ausgebildet ist, eine Flüssigkeit in Strömung zu versetzen;
   einem Sauganschluss (C1), welcher ein Ansaugen einer Flüssigkeit in die Pumpenkammer (P1) ermöglicht;
   einem Auslassanschluss (C2), welcher ein Austreten einer Flüssigkeit in der Pumpenkammer (P1) ermöglicht;
   einer Kolbenvorrichtung (7), welche zum Hin- und Herbewegen in der Pumpenkammer (P1) durch eine Antriebsvorrichtung ausgebildet ist und ein Ansaugen in die Pumpenkammer (P1) von dem Sauganschluss (C1) und ein Austreten der Flüssigkeit aus dem Auslassanschluss aufgrund der Hin- und Her-Bewegung ermöglicht;
   Ventilvorrichtungen (J1, J2), welche zwischen der Pumpenkammer (P1) und einem Strömungsweg an einer Sauganschlussseite und einem Strömungsweg an einer Auslassanschlussseite angeordnet sind, und welche in Abhängigkeit zu einem Wechsel in einem Flüssigkeitsdruck in der Pumpenkammer (P1) zu öffnen und zu schließen sind, welcher abhängig von der Hin- und Her-Bewegung der Kolbenvorrichtung (7) erzeugt wird, um eine Strömung der Flüssigkeit in einem geöffneten Zustand zu ermöglichen und in einem geschlossenen Zustand zu blockieren;
   **gekennzeichnet durch**
   eine Erfassungseinrichtung, welche ausgebildet ist, eine Zeitspanne zu erfassen, während welcher die Ventilvorrichtungen (J1, J2) sich in einem vorgegebenen Zyklus in einem offenen Zustand oder in einem geschlossenen Zustand befinden, und
   eine Vorrichtung (12), welche eine Rechenoperation durchführt, um einen volumetrischen Pumpenwirkungsgrad oder eine Strömungsrate basierend auf einem Vergleich zwischen der erfassten Zeitspanne, welche durch die Erfassungsvorrichtung erfasst wird, und einer Zeitspanne, während welcher die Ventilvorrichtungen (J1, J2) sich in dem vorgegebenen Zyklus in einem offenen Zustand oder in einem geschlossenen Zustand während eines reibungslosen Betriebszustandes der Kolbenpumpe befinden.

2. Kolbenpumpe nach Anspruch 1,
   **dadurch gekennzeichnet,**
   **dass** die Erfassungsvorrichtung eine Zeitspanne erfasst, während der die Ventilvorrichtung (J1) an der Sauganschlussseite und die Ventilvorrichtung (J2) an der Auslassanschlussseite in dem vorbestimmten Zyklus sich in einem geschlossenen Zustand befinden und die Rechenvorrichtung (12) eine Rechenoperation durchführt, um einen volumetrischen Pumpenwirkungsgrad oder eine Strömungsrate basierend einem Vergleich zwischen der Erfassungszeitspanne, welche durch die Erfassungsvorrichtung erfasst wird, und einer Zeitspanne zu erhalten, während der sich die Ventilvorrichtung (J1) an der Sauganschlussseite und die Ventilvorrichtung (J2) an der Auslassanschlussseite sich in einem geschlossenen Zustand in dem vorbestimmten Zyklus während eines reibungslosen Betriebszustandes der Kolbenpumpe befinden.

3. Kolbenpumpe nach Anspruch 1,
   **dadurch gekennzeichnet,**
   **dass** die Erfassungsvorrichtung eine Zeitspanne erfasst, während der die Ventilvorrichtung (J2) an der Auslassanschlussseite sich in dem vorbestimmten Zyklus in einem offenen Zustand befindet und die Rechenvorrichtung (12) eine Rechenoperation durchführt, um einen volumetrischen Pumpenwirkungsgrad oder eine Strömungsrate basierend einem Vergleich zwischen der Erfassungszeitspanne, welche durch die Erfassungsvorrichtung erfasst wird, und einer Zeitspanne zu erhalten, während der sich die Ventilvorrichtung (J2) an der Auslassanschlussseite sich in einem offenen Zustand in dem vorbestimmten Zyklus während eines reibungslosen Betriebszustandes der Kolbenpumpe befindet.

4. Kolbenpumpe nach einem der Ansprüche 1 bis 3,
   **dadurch gekennzeichnet,**
   **dass** die Flüssigkeit eine leitfähige Flüssigkeit enthält und die Erfassungsvorrichtung eine Sendeelektrodeneinheit und eine Empfangselektrodeneinheit, welche an der Pumpenkammer oder an dem Strömungsweg an der Sauganschlussseite beziehungsweise an dem Strömungsweg an der Auslassanschlussseite angeordnet sind, die so positioniert sind, dass die Ventilvorrichtung dazwischen angeordnet ist, und eine Berechnungsvorrichtung umfasst, die zum Berechnen einer Erfassungszeitspanne, während der die Ventilvorrichtung sich in einem geöffneten Zustand oder in einem geschlossenen Zustand befindet, basierend auf einer Zeitspanne der Stromanwendungszeit zwischen der Sendeelektrodeneinheit und der Empfangselektrodeneinheit (S1, S2) ausgebildet ist.

5. Kolbenpumpe nach Anspruch 4,
**dadurch gekennzeichnet,**
**dass** diese weiterhin aufweist:
eine Abschaltbestimmungsvorrichtung (13), die ausgebildet ist, zu bestimmen, ob sich die Ventilvorrichtung (J1, J2) in einem guten Abschaltzustand befindet oder nicht, und den Abschaltzustand basierend auf der Stromanwendung zwischen der Sendeelektrodeneinheit (Sa) und der Empfangselektrodeneinheit (S1, S2) zu überwachen.

6. Kolbenpumpe nach einem der Ansprüche 1 bis 5,
**dadurch gekennzeichnet,**
**dass** die Pumpenkammer (P1) ausgebildet ist mit einer flüssigkeitszuführseitigen Pumpenkammer (P1), welche mit einem flüssigkeitszuführseitigen Sauganschluss (C1) und einem flüssigkeitszuführseitigen Auslassanschluss (C2) in Verbindung steht, und
mit einer flüssigkeitsabflussseitigen Pumpenkammer (P2), welche mit einem flüssigkeitsabflussseitigen Sauganschluss (C3) und einem flüssigkeitsabflussseitigen Auslassanschluss (C4) in Verbindung steht, und
eine Anordnung aufweist, in welcher die Ventilvorrichtung (J1, J2, J3, J4) jeweils für den flüssigkeitszuführseitigen Sauganschluss, den flüssigkeitszuführseitigen Auslassanschluss, den flüssigkeitsabflussseitigen Sauganschluss und den flüssigkeitsabflussseitigen Auslassanschluss vorgesehen ist, wobei die Kolbenvorrichtung (7) zum Hin- und Herbewegen sowohl in der flüssigkeitszuführseitigen Pumpenkammer (P1) als auch der flüssigkeitsabflussseitigen Pumpenkammer (P2) ausgebildet ist, und
wobei Flüssigkeit in der flüssigkeitszuführseitigen Pumpenkammer (P1) und der flüssigkeitsabflussseitigen Pumpenkammer (P2) aus dem flüssigkeitszuführseitigen Auslassanschluss (C2) beziehungsweise dem flüssigkeitsauslassseitigen Auslassanschluss (C4) ausgestoßen wird.

7. Kolbenpumpe nach Anspruch 6,
**dadurch gekennzeichnet,**
**dass** zu einer Blutreinigungsvorrichtung (5) geführtes Dialysat in der flüssigkeitszuführseitigen Pumpenkammer (P1) fließt, aus der Blutreinigungsvorrichtung (5) herausgeführtes Dialysat in der flüssigkeitsabflussseitigen Pumpenkammer (P2) fließt und durch die Hin- und Herbewegung der Kolbenvorrichtung (7) Dialysat zu der Blutreinigungsvorrichtung (5) zuführbar und aus der Blutreinigungsvorrichtung (5) abführbar ist.

8. Kolbenpumpe nach Anspruch 6 oder 7,
**dadurch gekennzeichnet,**
**dass** die Rechenvorrichtung (12) einen volumetrischen Pumpenwirkungsgrad oder eine Strömungsrate in der flüssigkeitszuführseitigen Pumpenkammer (P1) und in der flüssigkeitsabflussseitigen Pumpenkammer (P2) erhält und ausgebildet ist, eine Flüssigkeitsleckage zwischen dem flüssigkeitszuführseitigen Auslassanschluss (C2) und dem flüssigkeitsabflussseitigen Sauganschluss (C3) basierend auf einem Vergleich zwischen dem volumetrischen Pumpenwirkungsgrad oder einer Strömungsrate in der flüssigkeitszuführseitigen Pumpenkammer (P1) und dem volumetrischen Pumpenwirkungsgrad oder der Strömungsrate in der flüssigkeitsabflussseitigen Pumpenkammer (P2) zu erfassen.

**Revendications**

1. Pompe alternative (1) comprenant :

une chambre de pompe (P1) qui est apte à laisser s'écouler un liquide ;
un orifice d'aspiration (C1) qui permet à un liquide d'être aspiré dans la chambre de pompe (P1) ;
un orifice de décharge (C2) qui permet à un liquide dans la chambre de pompe (P1) d'être déchargé ;
un dispositif alternatif (7) qui est adapté pour effectuer un mouvement alternatif dans la chambre de pompe (P1) par un dispositif d'entraînement, et permet à un liquide d'être aspiré dans la chambre de pompe (P1) depuis l'orifice d'aspiration (C1) et permet au liquide d'être déchargé depuis l'orifice de décharge (C2) sous l'effet du mouvement alternatif ;
des dispositifs de vanne (J1, J2) qui sont disposés entre la chambre de pompe (P1) et une voie d'écoulement sur un côté d'orifice d'aspiration et une voie d'écoulement sur un côté d'orifice de décharge, pouvant être ouverts et fermés en réponse à un changement de pression de liquide dans la chambre de pompe (P1), qui est produit en fonction du mouvement alternatif du dispositif alternatif (7), pour permettre au liquide de s'écouler dans un état ouvert et pour bloquer l'écoulement du liquide dans un état fermé ;

**caractérisé par**

un dispositif de détection qui est apte à détecter une période de temps au cours de laquelle les dispositifs de vanne (J1, J2) sont dans l'état ouvert ou dans l'état fermé dans un cycle prédéterminé ; et

un dispositif arithmétique (12) qui réalise une opération arithmétique pour obtenir un rendement volumétrique de pompe ou un débit sur la base d'une comparaison entre la période de temps de détection détectée par le dispositif de détection et une période de temps au cours de laquelle les dispositifs de vanne (J1, J2) sont dans l'état ouvert ou dans l'état fermé dans le cycle prédéterminé au cours d'un état opérationnel sans usure de la pompe alternative.

2. Pompe alternative selon la revendication 1,
**caractérisée en ce que**
le dispositif de détection détecte une période de temps au cours de laquelle à la fois le dispositif de vanne (J1) sur le côté d'orifice d'aspiration et le dispositif de vanne (J2) sur le côté d'orifice de décharge sont dans l'état fermé dans le cycle prédéterminé et le dispositif arithmétique (12) réalise une opération arithmétique pour obtenir un rendement volumétrique de pompe ou un débit sur la base d'une comparaison entre la période de temps de détection détectée par le dispositif de détection et une période de temps au cours de laquelle le dispositif de vanne (J1) sur le côté d'orifice d'aspiration et le dispositif de vanne (J2) sur le côté d'orifice de décharge sont dans l'état fermé dans le cycle prédéterminé au cours d'un état opérationnel sans usure de la pompe alternative.

3. Pompe alternative selon la revendication 1,
**caractérisée en ce que**
le dispositif de détection détecte une période de temps au cours de laquelle le dispositif de vanne (J2) sur le côté de décharge est dans l'état ouvert dans le cycle prédéterminé et le dispositif arithmétique (12) réalise une opération arithmétique pour obtenir un rendement volumétrique de pompe ou un débit sur la base d'une comparaison entre la période de temps de détection détectée par le dispositif de détection et une période de temps au cours de laquelle le dispositif de vanne (J2) sur le côté d'orifice de décharge est dans l'état ouvert dans le cycle prédéterminé au cours d'un état opérationnel sans usure de la pompe alternative.

4. Pompe alternative selon l'une quelconque des revendications 1 à 3,
**caractérisée en ce que**
le liquide contient un liquide conducteur et le dispositif de détection inclut une unité d'électrode émettrice et une unité d'électrode réceptrice qui sont disposées sur la chambre de pompe et respectivement sur la voie d'écoulement sur le côté d'orifice d'aspiration ou sur la voie d'écoulement sur le côté d'orifice de décharge, qui sont positionnées pour interposer le dispositif de vanne entre celles-ci et un dispositif de calcul qui est apte à calculer une période de temps de détection au cours de laquelle le dispositif de vanne est dans l'état ouvert ou dans l'état fermé sur la base d'une période de temps d'application de courant entre l'unité d'électrode émettrice et l'unité d'électrode réceptrice (S1, S2).

5. Pompe alternative selon la revendication 4,
**caractérisée en ce qu'**elle comprend en outre :
un dispositif de détermination de fermeture (13) qui est apte à déterminer si le dispositif de vanne (J1, J2) est ou non dans un bon état de fermeture et à surveiller l'état de fermeture sur la base de l'application de courant entre l'unité d'électrode émettrice (Sa) et l'unité d'électrode réceptrice (S1, S2).

6. Pompe alternative selon l'une quelconque des revendications 1 à 5,
**caractérisée en ce que**
la chambre de pompe (P1) est adaptée pour avoir une chambre de pompe de côté d'acheminement de liquide (P1) qui communique avec un orifice d'aspiration de côté d'acheminement de liquide (C1) et un orifice de décharge de côté d'acheminement de liquide (C2) et une chambre de pompe de côté d'évacuation de liquide (P2) qui communique avec un orifice d'aspiration de côté d'évacuation de liquide (C3) et un orifice de décharge de côté d'évacuation de liquide (C4), et a une configuration dans laquelle le dispositif de vanne (J1, J2, J3, J4) est prévu pour chacun de l'orifice d'aspiration de côté d'acheminement de liquide, de l'orifice de décharge de côté d'acheminement de liquide, de l'orifice d'aspiration de côté d'évacuation de liquide et de l'orifice de décharge de côté d'évacuation de liquide, le dispositif alternatif (7) est adapté pour effectuer un mouvement alternatif à la fois dans la chambre de pompe de côté d'acheminement de liquide (P1) et dans la chambre de pompe de côté d'évacuation de liquide (P2), et dans laquelle des liquides dans la chambre de pompe de côté d'acheminement de liquide (P1) et dans la chambre de pompe de côté d'évacuation de liquide (P2) sont déchargés respectivement de l'orifice de décharge de côté d'acheminement de liquide (C2) et de l'orifice de décharge de côté d'évacuation de liquide (C4).

7. Pompe alternative selon la revendication 6,
   **caractérisée en ce que**
   un dialysat qui est guidé vers un dispositif de purification de sang (5) s'écoule dans la chambre de pompe de côté d'acheminement de liquide (P1), un dialysat qui est guidé hors du dispositif de purification de sang (5) s'écoule dans la chambre de pompe de côté d'évacuation de liquide (P2), et le mouvement alternatif du dispositif alternatif (7) permet au dialysat d'être acheminé dans le dispositif de purification de sang (5) et d'être guidé hors du dispositif de purification de sang (5).

8. Pompe alternative selon la revendication 6 ou 7,
   **caractérisée en ce que**
   le dispositif arithmétique (12) obtient un rendement volumétrique de pompe ou un débit dans la chambre de pompe de côté d'acheminement de liquide (P1) et dans la chambre de pompe de côté d'évacuation de liquide (P2) et est apte à détecter une fuite de liquide entre l'orifice de décharge de côté d'acheminement de liquide (C2) et l'orifice d'aspiration de côté d'évacuation de liquide (C3) sur la base d'une comparaison entre un rendement volumétrique de pompe ou un débit dans la chambre de pompe de côté d'acheminement de liquide (P1) et le rendement volumétrique de pompe ou le débit dans la chambre de pompe de côté d'évacuation de liquide (P2).

[ Fig 1 ]

[ Fig 2 ]

[ Fig 3 ]

[ Fig 4 ]

EP 3 012 455 B1

[ Fig 5 ]

[ Fig 6 ]

[ Fig 7 ]

[ Fig 8 ]

[ Fig 9 ]

[ Fig 10 ]

(a)

(b)

(c)

[ Fig 11 ]

Output From Suction-side Integrating Circuit

Output From Discharge-side Integrating Circuit

Output From Suction-side Voltage Comparing Circuit

Output From Discharge-side Voltage Comparing Circuit

Output From Logic Circuit

[ Fig 12 ]

[ Fig 13 ]

[ Fig 14 ]

[ Fig 15 ]

**EP 3 012 455 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2505836 A1
- EP 0183295 A1
- EP 2550984 A1
- JP 2003284772 A
- JP 7174659 A